# EUROPÄISCHE PATENTSCHRIFT

(11) **EP 1 807 503 B1**
(45) Veröffentlichungstag und Bekanntmachung des Hinweises auf die Patenterteilung: **04.01.2012**
(21) Anmeldenummer: 05802887.9
(22) Anmeldetag: 02.11.2005
(51) Int. Cl.: C12M 3/00

(54) **VERFAHREN UND VORRICHTUNGEN ZUR BEARBEITUNG EINZELNER BIOLOGISCHER ZELLEN**
METHOD AND DEVICES FOR TREATING INDIVIDUAL BIOLOGICAL CELLS
PROCEDES ET DISPOSITIFS DE TRAITEMENT DE CELLULES BIOLOGIQUES INDIVIDUELLES

(30) Priorität: 05.11.2004 DE 102004053596
(43) Veröffentlichungstag der Anmeldung: 18.07.2007
(73) Patentinhaber: Fraunhofer-Gesellschaft zur Förderung der angewandten Forschung e.V., 80686 München (DE)
(72) Erfinder: FUHR, Günter, 13187 Berlin (DE); THIELECKE, Hagen, 66440 Blieskastel (DE)
(74) Vertreter: Hertz, Oliver
(86) Internationale Anmeldenummer: PCT/EP2005/011720
(87) Internationale Veröffentlichungsnummer: WO 2006/048256

(56) Entgegenhaltungen:
- WO-A-2004/074424
- DE-A1-102004 005 672
- DE-C1- 19 714 987
- DE-C1- 19 841 337
- US-A- 5 877 008
- US-A1- 2003 228 695
- US-A1- 2004 092 002
- DATABASE WPI Section Ch, Week 199703 Derwent Publications Ltd., London, GB; Class D16, AN 1997-028867 XP002362105 & JP 08 290377 A (PRIMA MEAT PACKERS LTD) 5. November 1996 (1996-11-05)

## Beschreibung

Die Erfindung betrifft Verfahren zur Bearbeitung einer biologischen Zelle mit den Merkmalen des Oberbegriffs von Anspruch 1. Des Weiteren betrifft die Erfindung Vorrichtungen zur Durchführung derartiger Verfahren und deren Verwendungen.

Aus der Zellbiologie sind mehrere Verfahren zur invasiven Manipulation biologischer Zellen bekannt. Beispielsweise ist bei der Einführung einer Elektrodenspitze in eine Zellmembran für die "patch clamp"-Technik oder bei der Einführung einer Kanüle in die Zelle zur Entnahme von Kernmaterial eine Unterbrechung der Zellmembran und/oder ein Eindringen in das Zellplasma erforderlich. Diese Techniken haben generell den Nachteil, dass mit dem invasiven Eingriff in die Zelle eine Unterbrechung im molekularen Verbund mindestens der Zellmembran verursacht wird, in deren Ergebnis nach der jeweiligen Manipulation die Zelle in der Regel nicht mehr lebensfähig ist. Um dennoch die Lebensfähigkeit zu erhalten, dürfen mit den herkömmlichen Techniken nur minimale Verletzungen der Zellmembran gebildet werden (zum Beispiel im Mikrometer-Bereich oder darunter). Dies stellt jedoch eine erhebliche Limitierung in der Anwendbarkeit der herkömmlichen Techniken dar.

Eine weitere invasive Zellmanipulation stellt die Fusion biologischer Zellen dar. Zur Durchführung einer Fusion werden die Zellmembranen benachbarter Zellen chemisch (z. B. durch bestimmte Substanzen.oder mit Viren) oder elektrisch (z. B. mit einem Hochspannungspuls, z.B. DE 198 41 337 C1) unterbrochen und so neu verschmolzen oder ausgeheilt, dass Zellbestandteile der einzelnen Zellen in einer gemeinsamen, geschlossenen Zellmembran enthalten ist. Die herkömmlichen Fusionstechniken besitzen den Nachteil, dass eine exakte und definierte Zellfusion zwischen zwei vorbestimmten Zellen nicht verfügbar ist. Bisher ist es erforderlich, zur Zellfusion eine Vielzahl von Zellen der jeweiligen chemischen oder elektrischen Behandlung auszusetzen und dann aus den behandelten Zellen die gewünschten Fusionsprodukte auszusortieren. Dabei können jedoch Fehlbehandlungen oder unerwünschte Mehrfachfusionen (z. B. Dreizell- oder Vierzellfusionen) auftreten. Dies ist jedoch mit einem erheblichen Verlust an ggf. seltenen oder wertvollen Zellen verbunden.

Aus WO 2004/074426 ist ein Verfahren zur schonenden Bearbeitung von Zellmaterial mit einer Vielzahl von biologischen Zellen bekannt, bei dem eine Sonde so durch das Zellmaterial bewegt wird, dass die Zellen von der Sonde verdrängt werden. Durch die in WO 2004/074426 beschriebene Steuerung der Sonde werden Verletzungen der Zellen vermieden. Invasive Eingriffe in die Zellen hingegen sind bei dieser Technik nicht vorgesehen.

Die Aufgabe der Erfindung ist es, verbesserte Verfahren zur Bearbeitung biologischer Zellen, insbesondere von einzelnen Zellen, Zellpaaren oder Zellgruppen bereitzustellen, mit denen die Nachteile der herkömmlichen Techniken überwunden werden können und die sich insbesondere durch einen schonenden Umgang mit dem Zellmaterial, eine hohe Zuverlässigkeit und Genauigkeit und eine hohe Ausbeute auszeichnen. Die Aufgabe der Erfindung ist es auch, verbesserte Vorrichtungen zur Umsetzung derartiger Verfahren bereitzustellen.

Diese Aufgaben werden mit Verfahren und Vorrichtungen mit den Merkmalen der Patentansprüche 1 und 17 gelöst. Vorteilhafte Ausführungsformen und Anwendungen der Erfindung ergeben sich aus den abhängigen Ansprüchen.

Verfahrensbezogen basiert die Erfindung auf der allgemeinen technischen Lehre, zur Bearbeitung einer biologischen Zelle mit einer Zellmembran, die insbesondere Zytoplasma und ein Zytoskelett umhüllt, die Zelle und ein Werkzeug relativ zueinander zu bewegen und durch eine Einwirkung des Werkzeugs die Zellmembran der Zelle zu unterbrechen, wobei sich während der Relativbewegung des Werkzeugs und der Zelle deren Zytoskelett in einem stabilen Gleichgewicht befindet. Vorteilhafterweise wird mit dem erfindungsgemäßen Verfahren ein invasiver Eingriff in die Zelle realisiert, während sich das Zytoskelett mechanisch in einem Zustand befindet, in dem es im wesentlichen frei von äußeren mechanischen Spannungen ist und in dem die strukturelle und funktionelle Beziehung zwischen der Zellmembran und inneren Zellbestandteilen und die Funktionsfähigkeit der inneren Zellbestandteile erhalten bleiben. Des weiteren befindet sich auch die Zellmembran während der Bewegung des Werkzeugs in dem stabilen Gleichgewicht. Während und nach dem invasiven Eingriff in die Zelle bleibt die Fähigkeit zum Stoffwechsel und damit die Lebensfähigkeit der Zelle oder wenigstens eines Teils der Zelle erhalten.

Die Relativbewegung des Werkzeugs und der Zelle erfolgt derart langsam, dass sich das Zytoskelett und die Zellmembran, die laufend einem molekularen Umordnungsprozess unterliegen, zu jedem Zeitpunkt an die geometrischen Bedingungen anpassen können, die durch die Oberfläche des Werkzeugs, insbesondere einer Arbeitsfläche des Werkzeugs, gegeben sind. Die Komponenten des Zytoskeletts, das insbesondere Filamente der Proteine Aktin und Tubolin und das Protein Myosin enthält und den zellkern und die übrigen Zellbestandteile relativ zueinander hält, haben während der Werkzeugbewegung ausreichend Zeit für eine Neuordnung auf der Zellmembran und/oder der Arbeitsfläche des Werkzeugs. Das erfindungsgemäße Verfahren ermöglicht daher vorteilhafterweise, dass während einer Zellbearbeitung die Zelle durch die Zellmembran oder die Arbeitsfläche des Werkzeugs geschlossen bleibt. Es wird ein invasiver Eingriff in die Zelle ermöglicht, ohne dass große, schädliche oder irreversible Zellöffnungen entstehen.

Die Erfinder haben festgestellt, dass einzelne adhärente Zellen, obwohl diese .grundsätzlich durch ihre Eigenbewegung langsamen Einwirkungen äußerer Sonden ausweichen können (siehe WO 2004/074426), durch ein langsam bewegtes Werkzeug bis zu einer Unterbrechung der Zellmembran deformiert werden können und dabei überraschenderweise lebensfähig bleiben. Von besonderem Vorteil ist es, dass die bearbeitete Zelle während des gesamten Zeitraums der Manipulation gegenüber der Umwelt abgeschlossen bleibt.

Als Unterbrechung der Zellmembran wird hier allgemein jede Störung des molekularen Verbundes der insbesondere aus Phospholipiden und Proteinen aufgebauten Membran verstanden, bei der Moleküle, die zunächst in der intakten Membran benachbart angeordnet waren, durch die äußere Einwirkung des Werkzeugs voneinander getrennt werden. Die Unterbrechung der Zellmembran ergibt eine Öffnung der Zellmembran, an der die inneren Zellbestandteile mit der Arbeitsfläche des Werkzeugs oder anderen Materialien in der Zellumgebung, wie zum Beispiel anderen Zellen oder Zellbestandteilen oder anderen Substanzen in Kontakt gelangt.

Mit der Relativbewegung des Werkzeugs und der Zelle wird hier jede Ortsänderung des Werkzeugs und/oder der Zelle bezeichnet, die zu einer Änderung der Relativkoordinaten des Werkzeugs und der Zelle führt. Da das erfindungsgemäße Verfahren vorzugsweise außerhalb des Körpers eines Organismus durchgeführt wird und die zu bearbeitende Zelle vorzugsweise adhärent auf einem Substrat angeordnet ist, wird die Relativbewegung durch wenigstens eine der folgenden Bewegungen erreicht: Bewegung des Werkzeugs, Bewegung der Zelle, insbesondere mit dem Substrat, und Bewegung von beiden Teilen. Die Bewegung des Werkzeugs kann insbesondere zusätzlich zu der gewünschten Verdrängungs- und Deformationsbewegung hin zu der Zelle mit einer Nachführungsbewegung überlagert sein, mit der eine Eigenbewegung des Zelle auf dem Substrat kompensiert wird. Wenn im folgenden in der Regel auf die Bewegung des Werkzeugs Bezug genommen wird, sind alle hier genannten anderen Bewegungen, insbesondere auch des Substrates eingeschlossen.

Gemäß einer bevorzugten Ausführungsform der Erfindung erfolgt die Bewegung des Werkzeugs mit einer Geschwindigkeit, die kleiner oder gleich der Umordnungsgeschwindigkeit des Zytoskeletts ist. Vorteilhafterweise wird damit der o. g. Gleichgewichtszustand der Zelle sichergestellt. Mit der Umordnungsgeschwindigkeit wird hier die Geschwindigkeit bezeichnet, mit der sich Teile des Zytoskeletts, wie zum Beispiel charakteristische Filamentstrukturen aufgrund des ständig ablaufenden molekularen Umordnungsprozesses der Zelle bewegen. Die Umordnungsgeschwindigkeit entspricht bspw. der Geschwindigkeit, mit der sich bei einer adhärent angeordneten Zelle ein äußerer Rand des Zytoskeletts relativ zu einem Substrat bewegt. Die Umordnungsgeschwindigkeit ist eine zellspezifische Größe, die in einem Vorversuch problemlos durch eine Messung ermittelt werden kann (siehe z. B. B. Alberts et al. in "Molekularbiologie der Zelle", Wiely VCH Verlag, Weinheim, 2004, P. Geggier et al. in "Appl. Phys. A", Bd. 68, 1999, S. 505 - 513; A. R. Bausch et al. in "Biophys. J.", Bd. 76, 1999, S. 573 - 579, T. J. Mitchison et al. in "Cell", Bd. 84, 1996, S. 371 - 379).

Vorteile für die Einstellung der Werkzeuggeschwindigkeit können sich gemäß einer weiteren Ausführungsform der Erfindung ergeben, wenn die Bewegung des Werkzeugs mit einer Geschwindigkeit erfolgt, die kleiner oder gleich einer Wanderungsgeschwindigkeit von adhärenten Zellen auf einer Oberfläche sind. Diese Wanderungsgeschwindigkeit kann in einem Vorversuch unter den konkreten Arbeitsbedingungen an der zu bearbeitenden Zelle ermittelt werden. Die Erfinder haben festgestellt, dass die physiologische Wanderungsgeschwindigkeit der Zelle unmittelbar mit der o. g. Umordnungsgeschwindigkeit des Zytoskeletts in Zusammenhang steht und so als Bezugsgröße für die Werkzeugbewegung verwendet werden kann.

Vorzugsweise wird das Werkzeug mit einer Geschwindigkeit bewegt, die kleiner als 500 µm/h, insbesondere kleiner als 300 µm/h ist. Vorteilhafterweise sind in diesem Geschwindigkeitsbereich die o. g. Bedingungen für die meisten interessierenden Zelltypen, wie z. B. Fibroblasten, Makrophagen oder Krebszellen erfüllt, so dass zum Beispiel auf Vorversuche oder Tabellenwerte verzichtet werden kann.

Wenn gemäß einer weiteren bevorzugten Ausführungsform der Erfindung die biologische Zelle auf der festen Oberfläche eines ebenen Substrats angeordnet ist und das Werkzeug relativ zu dem Substrat bewegt wird, können sich Vorteile für die Zusammenwirkung des Werkzeugs und des Substrates ergeben. Die feste Oberfläche bildet eine Auflage zur Erzeugung einer Gegenkraft während der Vortriebsbewegung des Werkzeugs.

Die Vortriebsbewegung des Werkzeugs relativ zu dem Substrat umfasst vorzugsweise wenigstens eine von zwei Phasen, durch welche die Strukturbildung während und nach der erfindungsgemäßen Zellbearbeitung positiv beeinflusst wird. In einer Annäherungsphase wird das Werkzeug zu dem Substrat bewegt und die Zelle zwischen dem Werkzeug und dem Substrat deformiert und eingeschnürt. In einer Translationsphase wird das Werkzeug im Wesentlichen parallel zur Oberfläche des Substrats bewegt. In dieser Phase wird die Umordnung der Zellmembran gefördert.

Die genannten Bewegungsarten entsprechend den Annäherungs- und Translationsphasen können erfindungsgemäß einzeln, in Kombination aufeinanderfolgend oder in Kombination gleichzeitig realisiert werden. Beispielsweise ist bei der Bewegung gemäß der Annäherungsphase (Verminderung des Werkzeug-Substrat-Abstandes) eine Trennung der Zelle in mindestens zwei Zellkörper wie bei einem Stanzvorgang vorgesehen. Die erfindungsgemäße Zellbearbeitung kann ferner beispielsweise allein eine Bewegung des Werkzeugs parallel zur Substratoberfläche umfassen. Hierzu wird das Werkzeug zunächst neben der zu bearbeitenden Zelle auf der Substratoberfläche aufgesetzt. Das Werkzeug berührt beim Aufsetzen oder nach einer ersten Vorschubbewegung einen Rand der Zelle und wird dann gemäß dem Verfahren der Erfindung durch die Zelle hindurchbewegt, wobei diese in bspw. zwei Zellkörper zerteilt wird. Vorteilhafterweise bildet die Substratoberfläche bei dieser Ausführungsform der Erfindung eine Führung für die Werkzeugbewegung.

Vorzugsweise werden während der Bewegung des Werkzeugs die Zellmembran und mit dieser Teile des Zytoplasmas und des Zytoskeletts einer Deformation, Stauchung und/oder Dehnung unterzogen. Vorteilhafterweise kann dadurch die Unterbrechung der Zellmembran mit der Bildung neuer Formen der Zelle oder Zellbestandteile nach dem invasiven Eingriff gefördert werden.

Besonders bevorzugt ist, wenn während und/oder nach dem invasiven Eingriff in die Zellmembran eine Heilung der Unterbrechung der Zellmembran erfolgt, so dass die Zellmembran geschlossen bleibt bzw. wird. Die Heilung kann als Spontanheilung erfolgen, indem sich die Zellmembran bei ruhendem Werkzeug unter der Wirkung der Oberflächenspannung schließt, oder durch eine gerichtete Bewegung des Werkzeugs gefördert werden. Vorteilhafterweise wird die Zelle durch die Heilung geschlossen und damit auch ohne den Kontakt mit der Arbeitsfläche weiterhin lebensfähig.

Wenn während der Unterbrechung und der folgenden Heilung der Zellmembran der Innenraum der Zelle von der Umgebung stofflich getrennt ist, kann eine unerwünschte oder nicht reproduzierbare Veränderung der Struktur und Funktion der Zelle, insbesondere des Stoffwechsels und des Erbmaterials vermieden werden, wobei dies vorteilhafterweise einen positiven Einfluss auf die weitere Verwendung der Zelle für Kultivierungs- oder Messzwecke hat.

Alternativ kann erfindungsgemäß vorgesehen sein, dass während der Unterbrechung ein Fremdstoff, wie zum Beispiel ein Markierungsstoff in die Zelle eingeschleust wird.

Weitere Ausführungsformen der Erfindung zeichnen sich dadurch aus, dass das Werkzeug nach der Bearbeitung der Zelle, z. B. nach der Trennung der Zelle in mindestens zwei Zellkörper weiter in Kontakt mit der Oberfläche des Substrats bleibt. Ein zur Trennung verwendetes Werkzeug ruht auf der Substratoberfläche, so dass vorteilhafterweise eine mechanische Wand zwischen den Zellkörpern gebildet wird.

Ein für die Anwendung des erfindungsgemäßen Verfahrens in der Zellbiologie besonders wichtiges Merkmal der Erfindung besteht darin, dass die Zellmembran nach der Heilung eine andere Form, Zusammensetzung und/oder Größe aufweist als vor der Bildung der Unterbrechung. Diese Veränderungen ermöglichen, dass Untersuchungen und/oder Veränderungen an der Zelle in definierter und reproduzierbarer Weise durchgeführt werden können.

Beispielsweise kann vorgesehen sein, dass die Zelle nach der Heilung in wenigstens zwei Zellkörper getrennt ist, die jeweils eine topologisch geschlossene Form aufweisen. Das erfindungsgemäße Verfahren führt zu einer Abschnürung der Zelle, so dass die getrennten Zellkörper gebildet werden. Die Zellkörper können als Startmaterialien für weitere Kultivierungen, Bearbeitungen oder Untersuchungen verwendet werden und bieten dabei den Vorteil, von einer gemeinsamen Vorläuferzelle abgetrennt zu sein. Besonders bevorzugt ist, wenn einer der getrennten Zellkörper kernlos ist. In diesem Fall ist der jeweils andere Zellkörper mit dem Zellkern voll lebensfähig, während der kernlose Zellkörper ebenfalls Stoffwechselaktivitäten zeigen kann, so lange ATP von den in diesem enthaltenen Mitochondrien produziert wird. Der kernlose Zellkörper kann anschließend beispielsweise einer Untersuchung unterzogen werden, um stoffliche Eigenschaften des anderen Zellkörpers zu ermitteln, ohne direkt auf diesen zuzugreifen.

Alternativ kann beispielsweise vorgesehen sein, dass die Zellmembran nach der Heilung mit einer fremden Zellmembran einer weiteren biologischen Zelle mindestens einen topologisch geschlossenen Zellkörper bildet. Vorteilhafterweise kann damit die Oberfläche der (ersten) bearbeiteten Zelle mit Stoffen oder Strukturen der Oberfläche der zweiten Zelle modifiziert werden, beispielsweise um bestimmte Antigene oder Markierungsstoffe auf der ersten Zelle zu präsentieren. Besonders bevorzugt ist hierbei die Fusion oder Paarbildung von Zellen, bei der die erste Zelle mit mindestens einer weiteren Zelle fusioniert wird. Die erfindungsgemäße Zellfusion hat gegenüber den herkömmlichen Fusionsmethoden den besonderen Vorteil, dass die o. g. Fehlbehandlungen oder ggf. unerwünschten Mehrfachfusionen vermieden werden können und gezielt mit praktisch 100-%iger Ausbeute selbst seltene oder besonders wertvolle Zellen, wie zum Beispiel Stammzellen, fusioniert werden können. Eine bevorzugte Anwendung der erfindungsgemäßen Zellfusion ist auch bei der Hybridomatechnik zur Herstellung monoklonaler Antikörper gegeben.

Weitere Vorteile für die Steuerbarkeit und Reproduzierbarkeit der Modifizierung der ersten Zelle mit Material von einer fremden Zelle, insbesondere bei der Zellfusion, können sich ergeben, wenn die Zellen während der Bearbeitung mit dem Werkzeug auf einem Substrat einander zumindest teilweise überlappend und insbesondere übereinander angeordnet sind.

Vorrichtungsbezogen wird die o. g. Aufgabe durch einen Zellmanipulator gelöst, der insbesondere ein Werkzeug zur Bearbeitung von einzelnen biologischen Zellen mit mindestens einer Arbeitsfläche, mit der die Zellmembran der Zelle lokal selektiv deformierbar ist, und eine Antriebseinrichtung aufweist, mit der das Werkzeug so bewegt werden kann, dass sich das Zytoskelett der Zelle während der Einwirkung des Werkzeugs auf die Zelle in einem mechanisch stabilen Gleichgewicht befindet. Vorteilhafterweise können mit dem Zellmanipulator genau und reproduzierbar invasive Eingriffe an der Zelle entsprechend dem erfindungsgemäßen Verfahren realisiert werden.

Die Antriebseinrichtung des erfindungsgemäßen Zellmanipulators ist insbesondere für Vortriebsgeschwindigkeiten des Werkzeugs im Bereich von 0.1 µm/h bis 500 µm/h eingerichtet ist. Hierzu ist vorzugsweise ein piezoelektrischer Antrieb oder einen Magnetantrieb vorgesehen, die eine genaue Einstellung derart langsamer Werkzeugbewegungen erleichtern.

Gemäß der Erfindung weist die Arbeitsfläche des Werkzeugs eine charakteristische Größe aufweist, die geringer als die Größe der Zelle, insbesondere geringer als die laterale Ausdehnung einer Zelle auf einem Substrat ist. Die charakteristische Größe ist kleiner als 10 µm. Mit der charakteristische Größe der Arbeitsfläche wird hier die Ausdehnung der Kontaktfläche bezeichnet, die sich bei einer Berührung des Werkzeugs mit der Zelle vor der Bewegung des Werkzeugs bildet. Durch die Miniaturisierung der Arbeitsfläche kann der Zellmanipulator vorteilhafterweise für die verschiedensten Zelltypen verwendet werden. Besonders bevorzugt weist die Arbeitsfläche des Werkzeugs eine charakteristische Größe auf, die geringer als 1 µm ist. Vorteilhafterweise können auch derart kleine Arbeitsflächen ausreichend stabil bereitgestellt werden, da die Bewegung des Werkzeugs beim erfindungsgemäßen Verfahren im Wesentlichen kräftefrei erfolgt.

Vorteilhafterweise kann das Werkzeug und insbesondere dessen Arbeitsfläche in Abhängigkeit von der konkreten Anwendung der Erfindung optimiert werden. Gemäß einer Variante ist die Arbeitsfläche so gebildet, dass das Werkzeug eine gerade Seitenkante aufweist, die im Betrieb parallel zur Oberfläche des Substrats ausgerichtet ist. Die Anpassung der Form der Seitenkante an die Substratoberfläche ist für eine effektive Zusammenwirkung von Werkzeug und Substrat von Vorteil. Gemäß einer alternativen Variante kann die Arbeitsfläche eine Strukturierung mit Vorsprüngen, wie z. B. spitzenförmigen Erhebungen aufweisen. Die Strukturierung an der Arbeitsfläche des Werkzeugs erleichtert eine Fixierung der Zelle auf dem Substrat während der Zellbearbeitung. Schließlich kann die Arbeitsfläche eine komplexe Form mit mehreren Teilflächen aufweisen, die eine Zerlegung der Zelle in mehr als zwei Teile ermöglichen. Vorteilhafterweise kann die Arbeitsfläche z. B. eine Kreuzform bilden, um eine Zelle in vier Zellkörper zu zerlegen.

Wenn das Werkzeug zumindest entlang der Arbeitsfläche eine Beschichtung trägt, die ein Anhaften von biologischen Zellen behindert, ergeben sich Vorteile für die Steuerbarkeit der Werkzeugbewegung, da durch die Beschichtung der Arbeitsfläche eigenständige Zellbewegungen der Zelle behindert werden.

Gemäß einer weiteren Modifizierung der Erfindung ist der Zellmanipulator zusätzlich mit einer Positioniereinrichtung, mit der wenigstens eine der Komponenten Antriebseinrichtung und ein Substrat mit der Zelle beweglich ist, und/oder einer Sensoreinrichtung ausgestattet, die der Erfassung der Position des Werkzeugs und/oder der zu bearbeitenden Zelle dient.

Ein erweiterter Anwendungsbereich des Zellmanipulators kann vorteilhafterweise erreicht werden, wenn zwei oder mehr Werkzeuge vorgesehen sind, die synchron mit einer gemeinsamen Antriebseinrichtung oder separat mit getrennten Antriebseinrichtungen bewegt werden.

Einen unabhängigen Gegenstand der Erfindung stellt die Verwendung des erfindungsgemäßen Verfahrens oder des erfindungsgemäßen Zellmanipulators zur Zellfusion dar.

Weitere bevorzugte Anwendungen der Erfindung sind bei der Diagnose oder Charakterisierung einzelner Zellen, wie z. B. bei der stofflichen Analyse von Bestandteilen des Zytoplasmas oder bei einer Analyse des genetischen Materials in der einzelnen Zelle, oder bei Tests der Wechselwirkung der einzelnen Zelle mit fremden Stoffen, wie bspw. bei der Untersuchung von pharmakologischen Wirkstoffen gegeben. Für die Testanwendung ist von besonderem Vorteil, dass die zunächst einheitliche Zelle in verschiedene Teile getrennt werden kann, die jedoch auch nach der Trennung ein physiologisch homogenes System darstellen und verschiedenen Test- und Referenzstoffen ausgesetzt werden können.

Weitere Einzelheiten und Vorteile der Erfindung werden im Folgenden unter Bezug auf die beigefügten Zeichnungen beschrieben. Es zeigen:
- Figuren 1A bis 1D:: Illustrationen von Phasen der erfindungsgemäßen Bearbeitung einer biologischen Zelle;
- Figuren 2A bis 2C:: Illustrationen verschiedener Ausführungsformen der erfindungsgemäßen Bearbeitung biologischer Zellen;
- Figur 3:: eine schematische Darstellung einer Ausführungsform einer erfindungsgemäßen Vorrichtung zur Bearbeitung biologischer Zellen;
- Figur 4:: schematische Illustrationen verschiedener Werkzeuggestaltung;
- Figuren 5 bis 8:: weitere Illustrationen von Ausführungsformen der erfindungsgemäßen Bearbeitung biologischer Zellen; und
- Figur 9:: fotografische Abbildungen, die mehrere Phasen einer erfindungsgemäßen Trennung einer Zelle in zwei Zellkörper zeigen.

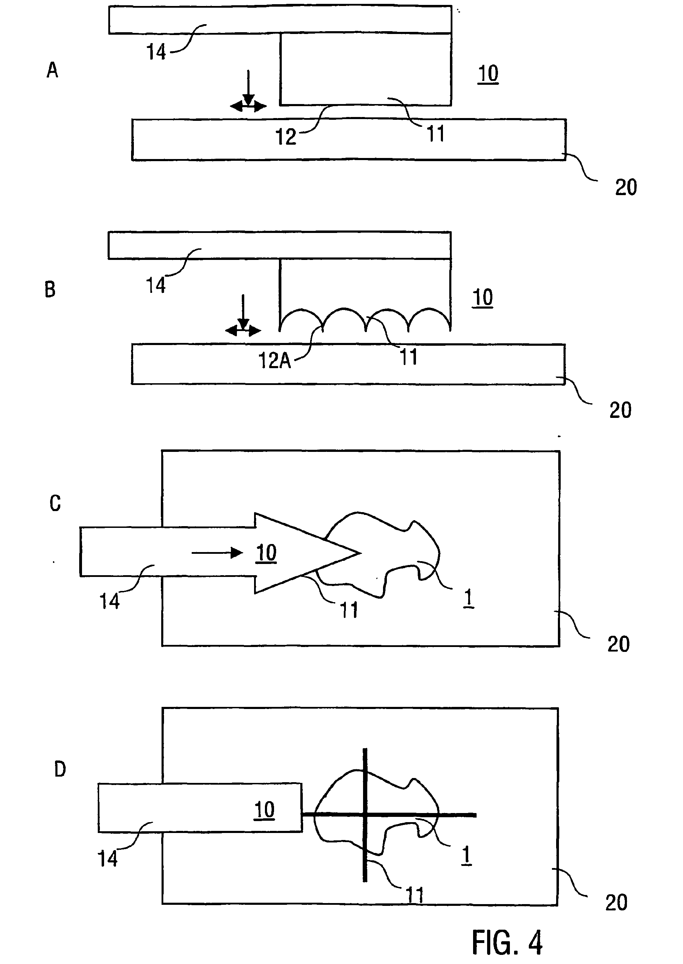

Die Erfindung wird im Folgenden unter beispielhaftem Bezug auf bevorzugte Ausführungsformen beschrieben, welche die Verwendung eines Werkzeugs illustrieren, das als Arbeitsfläche eine keil- oder kegelstumpfförmige Schneide aufweist. Es wird betont, dass die Umsetzung der Erfindung nicht auf die gezeigten Beispiele beschränkt, sondern entsprechend den oben genannten Merkmalen abgewandelt auch mit anderen Werkzeugformen realisierbar ist (siehe z. B. Figur 4). Des Weiteren wird betont, dass die biologischen Zellen in den beigefügten Zeichnungen lediglich schematisch gezeigt sind und in der Praxis je nach Zelltyp andere Formen aufweisen können. Einzelheiten der herkömmlichen Techniken zur Zellkultivierung und zur Beobachtung von Zellkulturen z. B. mit einem Mikroskop, werden im Einzelnen nicht beschrieben, da sie an sich bekannt sind.

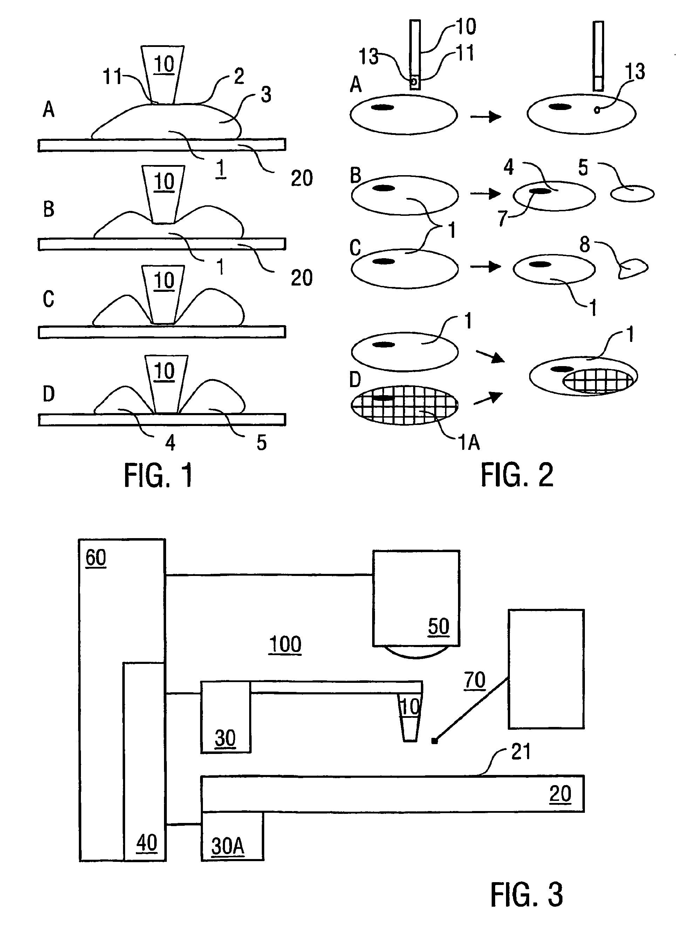

Die Schrittfolge einer ersten Ausführungsform des erfindungsgemäßen Verfahrens.ist schematisch in den Teilbildern A bis D von Figur 1 illustriert. Gemäß Figur 1A ist eine biologische Zelle 1 auf einem Substrat 20 angeordnet. Die Zelle 1 umfasst eine Zellmembran 2, die insbesondere das Zytoskelett 3 umhüllt, das den Zellkern und weitere Zellbestandteile (nicht dargestellt) umhüllt. Das Substrat 20 ist bspw. ein Kulturträger, z. B. aus Glas oder Kunststoff, der in einem Kulturgefäß (nicht dargestellt) angeordnet oder als Teil von diesem gebildet ist. Das in schematischer Schnittansicht gezeigte Werkzeug 10 weist die Gestalt eines Keils auf, dessen in der Figur oberer, breiter Fuß über weitere mechanische Komponenten (siehe Fig. 3) mit einer Antriebseinrichtung verbunden ist und dessen unteres, freies Ende die Arbeitsfläche 11 zur Einwirkung auf die Zelle 1 bildet.

Bei der in Fig. 1 gezeigten Ausführungsform der Erfindung werden zunächst das Werkzeug 10 und die Zelle 1 relativ zueinander so ausgerichtet, dass die Arbeitsfläche 11 des Werkzeugs 10 die Zellmembran 2 von außen berührt (Fig. 1A). Diese gegenseitige Ausrichtung kann unter visueller Kontrolle in einem Mikroskop oder automatisiert z. B. mit einem optischen Sensor gesteuert werden. Anschließend wird das Werkzeug 10 mit einer Geschwindigkeit von 20 - 100 µm/h in senkrechter Richtung an das Substrat 20 angenähert, wobei die Zelle 1 deformiert wird (Fig. 1B). Die Deformation führt zur Bildung einer lokalen Vertiefung der Zelloberfläche. Die zunächst in dem sich verminderten Abstand zwischen der Arbeitsfläche 11 und dem Substrat 20 angeordneten Teile der Zellmembran und inneren Zellbestandteile werden mit der fortschreitenden Bewegung des Werkzeugs 10 zunehmend verdrängt. Während dieser Verdrängung befindet sich das Zytoskelett 3 in einem Gleichgewichtszustand. Dies bedeutet, dass Filamente des Zytoskeletts 3 während der Werkzeugbewegung Verbindungen mit der Zellmembran oder gegenseitige Verbindungen freigeben und in benachbarten Bereichen, in denen keine Verdrängung erfolgt, neu knüpfen. Diese Freigabe und darauffolgend neue Bindung des Zytoskeletts (Umordnung) erfolgt im Wesentlichen mit derselben Geschwindigkeit wie die natürliche Zytoskelettumordnung. Daher ergibt die Werkzeugbewegung zunächst lediglich eine Formänderung, die jedoch von der Zelle aufgrund der hohen Formvariabilität biologischer Zellen toleriert und kompensiert wird.

Wenn der Abstand zwischen dem Werkzeug 10 und dem Substrat 20 im Wesentlichen der doppelten Membrandicke entspricht, kommt es zur Abschnürung zwischen den Teilen der Zelle 1 auf den beiden Seiten des Werkzeugs 10 (Fig. 1C). Die Erfinder haben festgestellt, dass in dieser Phase der molekulare Verbund der Zellmembran 2 unterbrochen werden kann, ohne dass sich die Zelle gegenüber der Umgebung öffnet. Stattdessen führt die weitere Bewegung des Werkzeugs 10 nach der Abschnürung zu einer Verschmelzung der sich berührenden Abschnitte der Zellmembran 2, bis die Arbeitsfläche 11 des Werkzeugs 10 das Substrat 20 berührt und zwei getrennte Zellkörper 4, 5 gebildet sind (Figur 1D). Beide Zellkörper 4, 5 sind von einer topologisch geschlossenen Zellmembran umhüllt und jeweils lebensfähig, solange der Stoffwechsel im jeweiligen Zellkörper mit genügend Energie versorgt wird. Im weiteren Verfahren können in Abhängigkeit von der jeweiligen Anwendung an den Zellkörpern 4, 5 weitere Manipulationen, Kultivierungen oder Untersuchungen vorgenommen werden.

Figur 2 illustriert schematisch verschiedene Varianten invasiver Eingriffe an biologischen Zellen mit dem erfindungsgemäßen Verfahren. Gemäß Figur 2A ist ein Eindringen des Werkzeugs 10, auf dessen Arbeitsfläche 11 eine chemische Substanz 13 angeordnet ist, in die Zelle 1 vorgesehen. Die chemische Substanz 13 umfasst bspw. eine Markersubstanz für Fluoreszenzmessungen oder ein biologisch wirksames Makromolekül, wie z. B. einen DNS-Abschnitt. Das Werkzeug 10 wird mit der Substanz 13 durch die Zellmembran der Zelle 1 eingeführt und die Substanz 13 an einer gewünschten Position während einer Ruhephase des Werkzeugs 10 von diesem in das Zytoplasma abgegeben. Anschließend wird das Werkzeug 10 zurückgezogen. Der Ablauf gemäß Fig. 2A kann dahingehend abgewandelt sein, dass die Substanz 13 im Zellkern 7 abgelegt oder eine Substanz aus dem Zytoplasma oder dem Zellkern mit dem Werkzeug entnommen wird.

Figur 2B zeigt einen Ablauf analog zu Fig. 1, bei dem die Zelle 1 in zwei Zellkörper 4, 5 zerteilt wird, die jeweils eine topologisch geschlossene Form aufweisen. Der Zellkörper 4 enthält den Zellkern 7, während der Zellkörper 5 kernlos ist.

Figur 2C illustriert die Entnahme einer Probe 8 von der Zelle 1. Die Probe 8 umfasst bspw. einen Abschnitt der Zellmembran und/oder Teile des Zytoskeletts oder des Zytoplasmas, die mit dem Werkzeug von der Zelle 1 abgetrennt wurden, während der lebende Zustand der Zelle 1 erhalten bleibt.

Das Prinzip der erfindungsgemäßen Fusion zwischen verschiedenen Zellen 1, 1A ist in Fig. 2D schematisch gezeigt. Die Zellmembranen der aneinander angrenzend angeordneten Zellen 1, 1A werden mit dem erfindungsgemäßen Verfahren durch ein Werkzeug geöffnet und zu einem neuen Verbund verschmolzen, in dem ein Teil der fremden Zelle 1A in der ersten Zelle 1 aufgenommen (Fig. 2D) oder beide komplett verschmolzen wurden.

Die Komponenten eines erfindungsgemäßen Zellmanipulators 100 sind schematisch in Fig. 3 illustriert. Das Werkzeug 10 ist über eine mechanische Komponente wie z. B. einen Trägerstab, mit der Antriebseinrichtung 30 verbunden, die zur Einstellung der langsamen Verdrängungsbewegung des Werkzeugs 10 relativ zu einer Zelle (nicht dargestellt) auf dem Substrat 20 eingerichtet ist. Das Substrat 20 kann ebenfalls mit einer Antriebseinrichtung 30A ausgestattet sein, um einen langsamen Vortrieb relativ zum Werkzeug 10 zu bilden. Mit den Antriebseinrichtungen 30 und/oder 30A können ferner Zellbewegungen der Zelle auf dem Substrat 20 kompensiert werden.

Das Werkzeug 10 ist mit der Antriebseinrichtung 30 in allen drei Raumrichtungen, insbesondere senkrecht zur Oberfläche des Substrats 20 und in einer Ebene parallel zu dieser Oberfläche beweglich. Hierzu kann die Antriebseinrichtung 30 mehrere Einzelantriebe enthalten, die die Vortriebsbewegungen in die einzelnen Raumrichtungen bereitstellen. Die Antriebseinrichtungen 30, 30A und/oder die Einzelantriebe sind an sich bekannte piezoelektrische Antriebe, die für die Einstellung von Relativgeschwindigkeiten unterhalb von 300 µm/h eingerichtet sind.

Das Substrat 20 ist bspw. ein Kulturträger aus Glas oder Kunststoff. Auf der Oberfläche 21 des Substrats 20 kann eine strukturierte Beschichtung vorgesehen sein, die adhäsive Bereiche (Inseln), in denen Zellen bevorzugt anhaften, und nicht-adhäsive Bereiche aufweist, in denen die Zellen nicht oder mit verminderter Effektivität anhaften. Dies ermöglicht eine Bearbeitung von einer oder mehreren Zellen, während diese sich auf einer adhäsiven Insel befinden. Eine Zellwanderung wird durch die umgebenden nicht-adhäsiven Bereiche unterbunden, so dass eine gesonderte Nachführbewegung zur Kompensation der Zellwanderung vermieden werden kann.

Das Werkzeug 10 und/oder das Substrat 20 sind mit einer Positioniereinrichtung 40 relativ zueinander in allen drei Raumrichtungen verfahrbar. Die Positioniereinrichtung 40 ist ein an sich bekannter Stellantrieb, mit dem eine Positionierung des Werkzeugs 10 relativ zu der zu bearbeitenden Zelle bereitgestellt wird.

Das Bezugszeichen 50 bezeichnet allgemein eine optische Sensoreinrichtung zur Überwachung der Positionierung und der Verdrängungsbewegung des Werkzeugs 10 relativ zur Zelle. Typischerweise ist die Sensoreinrichtung 50 Teil eines Leichtmikroskops, in dessen Strahlengang das Werkzeug 10 und das Substrat 20 angeordnet sind.

Die Antriebseinrichtung 30 und/oder 30A, die Positioniereinrichtung 40 und die optische Sensoreinrichtung 50 werden mit einer Steuereinrichtung 60 überwacht und gesteuert. Die Steuereinrichtung 60 ist bspw. ein Mikrokontroller oder in einem Steuerrechner enthalten.

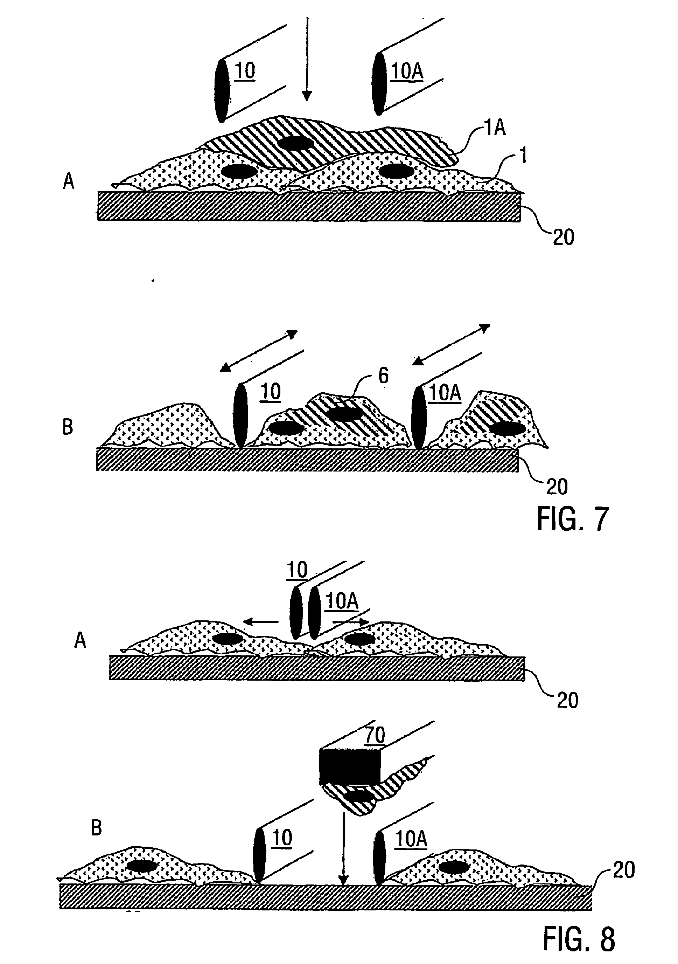

Das Bezugszeichen 70 verweist allgemein auf ein optional vorgesehenes weiteres Werkzeug, das ggf. unabhängig vom Werkzeug 10 betätigt werden kann (siehe Fig. 8).

Verschiedene Gestaltungen des Werkzeugs 10 des erfindungsgemäßen Zellmanipulators sind beispielhaft in Figur 4 gezeigt. Die Teilbilder A und B illustrieren in schematischer Seitenansicht jeweils das Werkzeug 10 mit der Arbeitsfläche 11 mit einem geringen Abstand von der Oberfläche des Substrats 20. Gemäß Teilbild A bildet die Arbeitsfläche 11 eine gerade Seitenkante 12, die bei paralleler Ausrichtung relativ zum Substrat 20 und senkrechter Annäherungsbewegung über ihre gesamte Länge die Oberfläche des Substrats 20 berührend auf diesem aufgesetzt werden kann. Gemäß Teilbild B ist die Arbeitsfläche 11 strukturiert, so dass keine glatte, gerade Seitenkante wie bei Teilbild A, sondern ein Gebilde mit einer Vielzahl von spitzenförmigen Vorsprüngen gegeben ist. Die Vorsprünge formen mehrere strukturierte, z. B. bogenförmige Seitenkantenabschnitte 12A, unter deren Einwirkung eine Zelle durch eine Translationsbewegung parallel zur Substratoberfläche in zwei Zellkörper getrennt werden kann.

Die Werkzeuge 10 der Teilbilder A und B werden vorzugsweise bei einer kombinierten Bewegung in den o. g. Annäherungs- und Translationsphasen verwendet. Die Länge der Seitenkante 12 beträgt bspw. 0.5 mm.

Die Teilbilder C und D der Figur 4 illustrieren in schematischer Draufsicht zwei weitere Gestaltungen des Werkzeugs 10. Gemäß Teilbild C ist eine keilförmige Arbeitsfläche 11 vorgesehen, mit der die Zelle 1 auf dem Substrat 20 bei Bewegung des Werkzeugs 10 parallel zur Substratoberfläche (Translationsphase) in zwei Zellkörper aufgetrennt werden kann. Gemäß Teilbild D umfasst das Werkzeug eine kreuzförmige Arbeitsfläche 11, mit der die Zelle 1 auf dem Substrat 20 im Verlauf einer Annäherungsbewegung in vier Teile getrennt werden kann.

Die Komponente 14 illustriert jeweils schematisch ein mechanisches Bauteil (z. B. einen Trägerstab), über den das Werkzeug 10 mit der Positioniereinrichtung 30 (siehe Figur 3) verbunden ist.

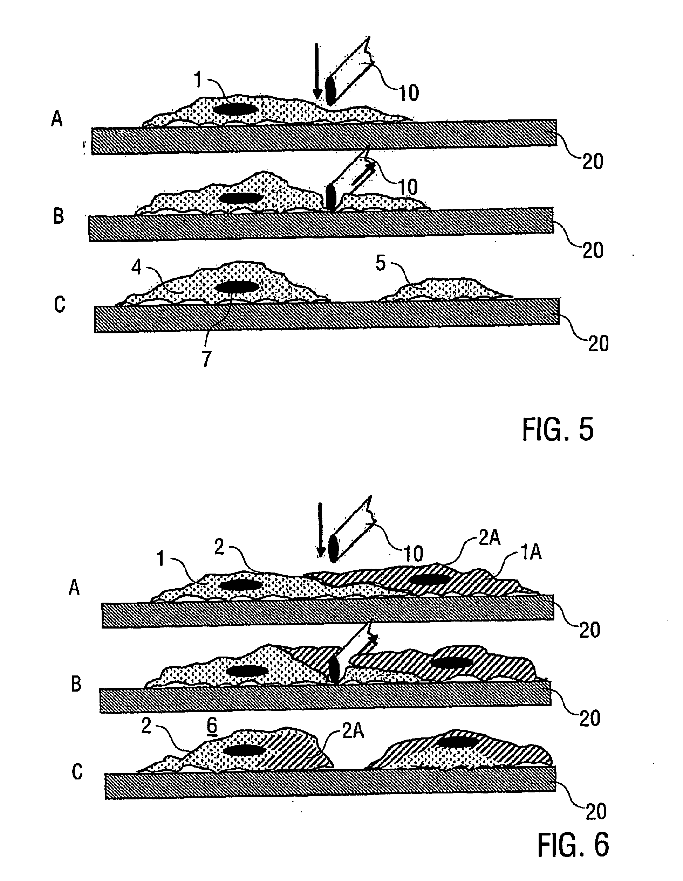

Die Figuren 5 bis 8 illustrieren schematisch experimentelle Ergebnisse, welche die Erfinder mit dem erfindungsgemäßen Verfahren erzielt haben. Figur 5 zeigt analog zu Fig. 1 und Fig. 2B mit weiteren Einzelheiten die Zerteilung einer Zelle 1 in zwei Zellkörper 4, 5. Gemäß Teilbild A ist eine adhärente Zelle 1, wie sie für in vitro-Kulturen typisch ist, auf dem Substrat 20 angeordnet. Die Zelle 1 ist z. B. eine Fibroblastenzelle oder eine adhärent auf einem Substrat angeordneten Stammzelle. Das Werkzeug 10 wird in Pfeilrichtung über der Zelle 1 mit der Positioniereinrichtung (40, siehe Fig. 3) ausgerichtet. Das in schematischer Perspektivansicht gezeigte Werkzeug 10 ist mit einer langgezogenen Schneide mit einem ellipsenförmigen Querschnitt ausgestattet, deren Oberfläche die Arbeitsfläche 11 bildet. Ein besonderer Vorteil der Erfindung ist es, dass bei zahlreichen Anwendungen die Form des Werkzeugs für die erfindungsgemäße Bearbeitung von Zellen keinen oder eine wesentlich geringere Bedeutung hat als bei den herkömmlichen Techniken mit schnellen Werkzeugbewegungen.

Die Länge der Schneide ist so gewählt, dass sich die Arbeitsfläche 11 über die gesamte Ausdehnung der Zelle 1 erstreckt. Die Breite der Schneide hingegen ist erheblich geringer als die Ausdehnung der adhärenten Zelle. Sie besitzt als charakteristische Größe der Arbeitsfläche bspw. einen Wert von 5 µm. Die Werkzeugbewegung umfasst zwei Phasen. Während der Annäherungsphase gemäß Figur 5A erfolgt die Werkzeugbewegung entlang einer Oberflächennormalen des Substrats 20 bspw. mit einer Geschwindigkeit im Bereich von 5 bis 50 µm/h. Wenn die Abschnürung der Zelle beidseitig des Werkzeugs 10 erreicht ist (Fig. 5B), folgt die Translationsphase der Werkzeugbewegung. Während dieser Phase wird das Werkzeug 10 parallel zur Oberfläche des Substrats 20 bewegt. Die Geschwindigkeit dieser Bewegung ist ebenfalls im Bereich von 5 bis 50 µm/h gewählt.

Nach dem Verschmelzen und Ausheilen der Zellmembranen der beiden Zellteile liegen die getrennten Zellkörper 4, 5 vor, die sich durch die eigenständige Zellwanderung sogar voneinander entfernen können (Fig. 5C). Der Zellkörper 4 enthält den Zellkern 7 und ist mit diesem weiterhin vital, bewegungsfähig und teilungsfähig. Der Zellkörper 5 kann zur Charakterisierung der ursprünglichen Zelle durch eine Analyse der Membrankomponenten des Zytoplasmas, von Zellorganellen und auch von genetischem Material im Zytoplasma, wie z. B. mRNS verwendet werden. Vorteilhafterweise können die zu untersuchenden Zellbestandteile quantitativ für die jeweilige Analyse ausreichend ohne einen Vitalitätsverlust des kernhaltigen Zellkörpers 4 abgetrennt werden.

Figur 6 illustriert einen analogen Verfahrensverlauf wie Fig. 5, wobei in diesem Fall jedoch zwei Zellen 1, 1A jeweils mit einer Zellmembran 2, 2A adhärent, sich gegenseitig überlappend auf dem Substrat 20 angeordnet sind. Derartige Überlappungen erfolgen in Zellkulturen häufig spontan, bspw. wenn eine hohe Zelldichte gegeben ist, oder wenn auf der Oberfläche des Substrats 20 eine sog. Feeder-Zellschicht vorhanden ist, wie es von der Kultivierungstechnik für Stammzellen bekannt ist.

Gemäß Figur 6A wird das Werkzeug 10 über dem Überlappungsbereich der Zellen 1, 1A positioniert, anschließend mit der o. g. Verdrängungsgeschwindigkeit abgesenkt und ggf. in einer Translationsphase parallel zur Oberfläche des Substrats 20 bewegt. Durch die invasive Einwirkung des Werkzeugs 10 werden die Zellmembranen 2, 2A der Zellen 1, 1A geöffnet und bei der anschließenden Heilung mit einer entsprechend veränderten Zusammensetzung und Form wieder verschlossen. Im Ergebnis sind in der Fusionszelle 6 Teile der Zellmembran 2A der Zelle 1A (schraffiert gezeichnet) in die Zellmembran der Zelle 1 (gepunktet gezeichnet) und umgekehrt eingebaut.

Mit dem in Figur 6 gezeigten Verfahren ist eine definierte paarweise Fusion biologischer Zellen möglich, wie sie bspw. bei der Ausstattung der ersten Zelle mit Antikörpern von der zweiten Zelle gewünscht ist.

Figur 7 illustriert ein weiteres Beispiel eines Fusionsvorgangs mit der Verwendung von zwei Werkzeugen 10, 10A. Auf dem Substrat 20 ist eine Monolage der Zellen 1 eines ersten Zelltyps vorgesehen, auf der eine oder mehrere Zellen 1A eines zweiten Zelltyps kultiviert werden. Diese Anordnung entspricht bspw. der Kultivierung von Stammzellen auf einer Feeder-Zellschicht oder der Kombination verschiedener Zelltypen beim sog. Tissue Engineering.

Analog zu den oben beschriebenen Verfahren werden die Werkzeuge 10, 10A in der Annäherungsphase langsam in das Zellmaterial gedrückt (Fig. 7A) und dann ggf. in der folgenden Translationsphase parallel zur Oberfläche des Substrats 20 bewegt (Fig. 7B). Durch die gleichzeitige Bewegung der Werkzeuge 10, 10A können neben der Fusion der Zellen 1, 1A im Bereich zwischen den Werkzeugen 10, 10A des Weiteren Teile des Zellmaterials von der Fusionszelle 6 separiert werden. Hierzu ist eine Bewegung der Werkzeuge 10, 10A senkrecht zur longitudinalen Ausdehnung der jeweiligen Arbeitsflächen und parallel zur Oberfläche des Substrats 20 vorgesehen.

Das in Figur 7 gezeigte Verfahren kann des Weiteren wie folgt modifiziert werden. Mit der optischen Sensoreinrichtung, insbesondere dem Mikroskop kann die Ausrichtung der Werkzeuge 10, 10A relativ zu den Zellen 1, 1A je nach den Anforderungen so eingestellt werden, dass in der Fusionszelle 6 beide Zellkerne oder lediglich ein Zellkern von einem der Zelltypen enthalten ist. Des Weiteren können die Zellen mit den Werkzeugen 10, 10A auf dem Substrat 20 zusammengeschoben werden, bevor die Fusion erfolgt. Schließlich können die Zellen und insbesondere die Fusionszelle 6 mit den Werkzeugen 10, 10A lateral auf dem Substrat 20 verschoben werden.

Figur 8 illustriert eine weitere komplexe Manipulation unter Verwendung von zwei Werkzeugen 10, 10A, die zunächst in eine Monolage der Zellen 1 auf dem Substrat 20 abgesenkt und anschließend lateral parallel zur Oberfläche des Substrats 20 und senkrecht zur longitudinalen Ausrichtung der Arbeitsflächen 11 auseinander geschoben werden (Fig. 8A). Bei diesem Vorgang wird eine Lücke in der Monoschicht (Zellrasen) geschaffen. Ein wichtiger Vorteil der Erfindung besteht darin, dass die Form dieser Lücke unabhängig von der Anordnung der Zellen ausschließlich durch die Positionierung und Absenkung der Werkzeuge festgelegt werden kann. Am umlaufenden Rand der Lücke werden im Unterschied zu herkömmlichen Ausschnittverfahren keine unerwünschten Wundränder, sondern geschlossene, ausgeheilte Membranformen gebildet. In die Lücke zwischen den Zellen 1 kann dann mit dem weiteren Werkzeug 70 (siehe auch Figur 3) eine oder mehrere Zellen oder ein künstliches Objekt eingesetzt werden. Das künstliche Objekt kann bspw. ein biologischer oder synthetischer Füllstoff, eine Sensoreinrichtung, ein Mikrosystem oder ein Gewebeteil (z. B. Nervenzellverbände oder Muskelzellverbände, Endothelien, Zusammensetzungen aus diesen) umfassen.

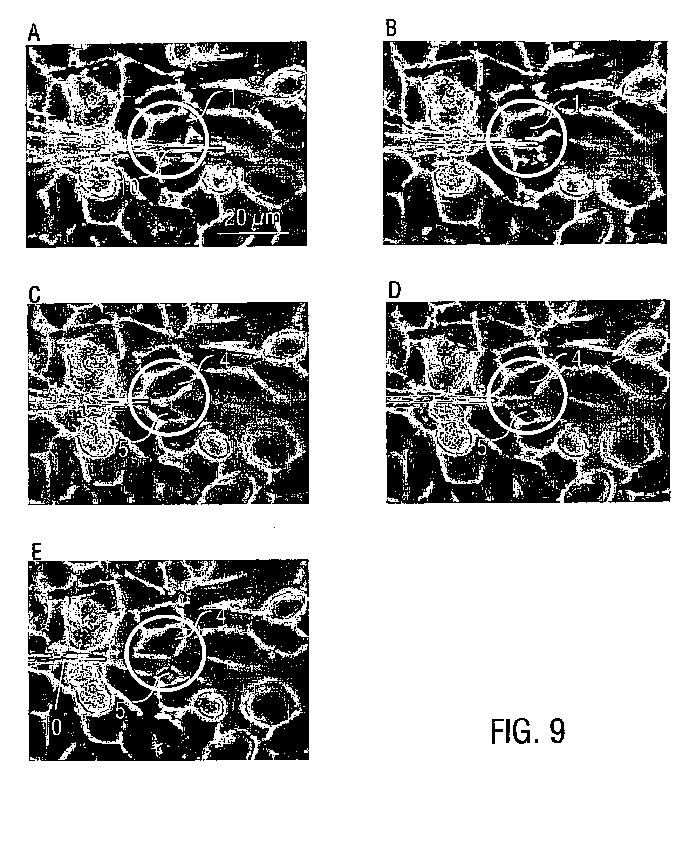

Die fotografischen Abbildungen A bis E in Figur 9 zeigen den Fortschritt der erfindungsgemäßen Trennung einer Zelle 1 mit dem Werkzeug 10 in zwei Zellkörper 4, 5 am Beispiel eines konkreten experimentellen Ergebnisses. Die Darstellung zeigt Fibroblastenzellen auf einem Substrat 20. Als Werkzeug 10 wird eine ausgezogene Glasspitze verwendet, deren Durchmesser am spitzen Ende rd. 2 µm beträgt. Gemäß Teilbild A wird das Werkzeug 10 zunächst neben der zu bearbeitenden Zelle 1 auf dem Substrat aufgesetzt. Anschließend wird das Werkzeug 10 gemäß der o. g. Translationsbewegung parallel zur Oberfläche des Substrats verschoben (in den Abbildungen nach links), wobei gemäß den Teilbildern C und D zunächst eine teilweise und im weiteren Bewegungsverlauf eine vollständige Trennung in die Zellkörper 4, 5 erfolgt. Teilbild E zeigt die weitere Beweglichkeit der Zellkörper 4, 5 nach der Trennung.

Die in der vorstehenden Beschreibung, den Ansprüchen und den Zeichnungen offenbarten Merkmale der Erfindung können sowohl einzeln als auch in Kombination für die Verwirklichung der Erfindung in ihren verschiedenen Ausgestaltungen von Bedeutung sein.

## Patentansprüche

1. Verfahren zur Bearbeitung einer biologischen Zelle (1, 1A), die ein von einer Zellmembran (2) umhülltes Zytoskelett (3) aufweist, mit den Schritten:
- gegenseitige Ausrichtung der biologischen Zelle (1, 1A) und eines Werkzeugs (10, 10A), so dass das Werkzeug (10, 10A) die biologische Zelle (1, 1A) berührt, und
- Bewegung des Werkzeugs (10, 10A) und der biologischen Zelle (1, 1A) relativ zueinander,
**dadurch gekennzeichnet, dass** - mit dem Werkzeug (10, 10A) eine Unterbrechung im molekularen Verbund der Zellmembran (2) der biologischen Zelle (1, 1A) gebildet wird, und
- die Bewegung des Werkzeugs (10, 10A) und der biologischen Zelle (1, 1A) relativ zueinander derart langsam erfolgt, dass sich das Zytoskelett und die Zellmembran der biologischen Zelle (1, 1A) zu jedem Zeitpunkt an geometrische Bedingungen anpassen können, die durch eine Arbeitsfläche des Werkzeugs (10, 10A) gegeben sind, so dass während der Bewegung des Werkzeugs (10, 10A) das Zytoskelett (3) der biologischen Zelle (1, 1A) einen Gleichgewichtszustand aufweist.

2. Verfahren nach Anspruch 1, bei dem die Bewegung des Werkzeugs (10, 10A) mit einer Geschwindigkeit erfolgt, die kleiner oder gleich einer Umordnungsgeschwindigkeit des Zytoskeletts (3) ist.

3. Verfahren nach Anspruch 1 oder 2, bei dem die Bewegung des Werkzeugs (10, 10A) mit einer Geschwindigkeit erfolgt, die kleiner oder gleich einer Wanderungsgeschwindigkeit von adhärenten Zellen auf einer Oberfläche sind.

4. Verfahren nach Anspruch 2 oder 3, bei dem die Bewegung des Werkzeugs (10, 10A) mit einer Geschwindigkeit erfolgt, die kleiner als 500 µm/h ist.

5. Verfahren nach mindestens einem der vorhergehenden Ansprüche, bei dem die biologische Zelle (1, 1A) auf einem Substrat (20) angeordnet ist und das Werkzeug (10, 10A) relativ zu dem Substrat (20) bewegt wird.

6. Verfahren nach Anspruch 5, bei dem die Bewegung des Werkzeugs (10, 10A) relativ zu dem Substrat (20) eine Annäherungsphase, bei welcher der Abstand des Werkzeugs (10, 10A) relativ zu dem Substrat (20) verringert wird, und/oder eine Translationsphase umfasst, bei welcher das Werkzeug (10, 10A) parallel zur Oberfläche des Substrats (20) verschoben wird.

7. Verfahren nach mindestens einem der vorhergehenden Ansprüche, bei dem während der Bewegung die Zellmembran (2) mit dem Werkzeug (10, 10A) einer Deformation, Stauchung und/oder Dehnung unterzogen wird.

8. Verfahren nach mindestens einem der vorhergehenden Ansprüche, mit dem weiteren Schritt:
- Heilung der Unterbrechung, so dass die Zellmembran (2) geschlossen wird, wobei die Heilung eine Spontanheilung, bei der sich die Zellmembran bei ruhendem Werkzeug unter der Wirkung der Oberflächenspannung schließt, umfasst oder durch eine gerichtete Bewegung des Werkzeugs gefördert wird.

9. Verfahren nach Anspruch 8, bei dem während der Bildung und der folgenden Heilung der Unterbrechung der Zellmembran (2) eine Trennung eines Innenraumes der Zelle (1, 1A) von der Umgebung-gegeben ist.

10. Verfahren nach Anspruch 8 oder 9, bei dem die Zellmembran (2) nach der Heilung eine andere Form, Zusammensetzung und/oder Größe besitzt als vor der Bildung der Unterbrechung.

11. Verfahren nach Anspruch 10, bei dem die Zelle (1, 1A) nach der Heilung in zwei Zellkörper (4, 5) getrennt ist, die jeweils eine topologisch geschlossene Form aufweisen.

12. Verfahren nach Anspruch 9, bei dem einer der getrennten Zellkörper (5) kernlos ist.

13. Verfahren nach Anspruch 12, bei dem der kernlose Teil (5) einer Untersuchung unterzogen wird.

14. Verfahren nach Anspruch 10, bei dem die Zellmembran (2) nach der Heilung mit einer fremden Zellmembran (2A) einer weiteren biologischen Zelle (1A) mindestens einen topologisch geschlossenen Zellkörper bildet.

15. Verfahren nach Anspruch 10, bei dem die biologische Zelle (1) mit mindestens einer weiteren biologischen Zelle (1A) zu einer Fusionszelle (6) fusioniert wird.

16. Verfahren nach Anspruch 15, bei dem die Zellen (1, 1A) während der Fusion auf einem Substrat (20) übereinander angeordnet sind.

17. Zellmanipulators (100), insbesondere zur Bearbeitung von biologischen Zellen (1, 1A), der umfasst:
- ein Substrat (20) zur Aufnahme einer Zelle (1, 1A),
- ein Werkzeug (10, 10A) zur Bearbeitung der biologischen Zelle (1, 1A), und
- eine Antriebseinrichtung (30, 30A) zur Bewegung des Werkzeugs (10, 10A) relativ zum Substrat (20),
**dadurch gekennzeichnet, dass**
- das Werkzeug (10, 10A) eine Arbeitsfläche (11) aufweist, unter deren Wirkung die Zellmembran (2) der biologischen Zelle (1, 1A) lokal selektiv deformierbar ist, wobei
- die Arbeitsfläche (11) des Werkzeugs (10, 10A) eine charakteristische Größe aufweist, die geringer als 10 µm ist.

18. Zellmanipulator nach Anspruch 17, bei dem die Arbeitsfläche (11) des Werkzeugs (10, 10A) eine Beschichtung (12) trägt die ein Anhaften von Zellen behindert.

19. Zellmanipulator nach mindestens einem der Ansprüche 17 oder 18, bei dem die Antriebseinrichtung (30, 30A) zur Bewegung des Werkzeugs (10, 10A) mit Geschwindigkeiten im Bereich von 0.1 µm/h bis 500 µm/h eingerichtet ist.

20. Zellmanipulator nach mindestens einem der Ansprüche 17 bis 19, bei dem die Antriebseinrichtung (30, 30A) einen piezoelektrischen Antrieb oder einen Magnetantrieb aufweist.

21. Zellmanipulator nach mindestens einem der Ansprüche 17 bis 20, der eine Positioniereinrichtung (40) zur Positionierung der Antriebseinrichtung (30, 30A) und/oder eines Substrats (20) mit der biologischen Zelle (1, 1A) aufweist.

22. Zellmanipulator nach mindestens einem der Ansprüche 17 bis 21, der eine Sensoreinrichtung (50) zur Erfassung der Position des Werkzeugs (10, 10A) und/oder der biologischen Zelle (1, 1A) aufweist.

23. Zellmanipulator nach mindestens einem der Ansprüche 17 bis 22, der mindestens zwei Werkzeuge (10, 10A) aufweist.

24. Zellmanipulator nach mindestens einem der Ansprüche 17 bis 23, bei dem das Werkzeug (10, 10A) eine gerade Seitenkante (12) oder strukturierte Seitenkantenabschnitte (12A) aufweist.

25. Verwendung eines Verfahrens oder eines Zellmanipulators nach mindestens einem der vorhergehenden Ansprüche zur:
- Fusion biologischer Zellen, und/oder
- Diagnose oder Charakterisierung einzelner Zellen.

## Claims

1. A method for treating a biological cell (1, 1A) that has a cytoskeleton enclosed by a cell membrane (2), with the steps:
- mutual orientation of the biological cell (1, 1A) and of a tool (10, 10A) so that the tool (10, 10A) touches the biological cell (1, 1A), and
- movement of the tool (10, 10A) and of the biological cell (1, 1A) relative to each other,
**characterized in that**
- a disruption is formed in the molecular compound of the cell membrane (2) of the biological cell (1, 1A) using the tool (10, 10A), and
- the movement of the tool (10, 10A) and the biological cell (1, 1A) relative to each other is so slow, that the cytoskeleton and the cell membrane of the biological cell (1, 1A) at every time can be adapted to the geometrical conditions, which are provided by a work surface of the tool (10, 10A), so that during the movement of the tool (10, 10A) the cytoskeleton (3) of the biological cell (1, 1A) is in a state of equilibrium.

2. The method according to Claim 1, in which the movement of the tool (10, 10A) takes place at a rate that is less than or equal to a rearrangement rate of the cytoskeleton (3).

3. The method according to Claim 1 or 2, in which the movement of the tool (10, 10A) takes place at a rate that is less than or equal to a migration rate of adherent cells on a surface.

4. The method according to Claim 2 or 3, in which the movement of the tool (10, 10A) takes place at a rate that is less than 500 µm/h.

5. The method according to at least one of the preceding claims, in which the biological cell (1, 1A) is arranged on a substrate (20) and the tool (10, 10A) is moved relative to the substrate (20).

6. The method according to Claim 5, in which the movement of the tool (10, 10A) relative to the substrate (20) comprises an approach phase in which the distance of the tool (10, 10A) relative to the substrate (20) is reduced, and/or a translation phase in which the tool (10, 10A) is shifted parallel to the surface of the substrate (20).

7. The method according to at least one of the preceding claims, in which the cell membrane (2) is subjected during the movement to a deformation, compression and/or elongation by the tool (10, 10A).

8. The method according to at least one of the preceding claims, with the further step:
- healing of the disruption so that the cell membrane (2) is closed, wherein the healing comprises a spontaneous healing, at which the cell membrane is closed with the resting tool under the effect of surface tension, or is stimulated by a directed movement of the tool.

9. The method according to claim 8, in which there is a separation of an inner space of the cell (1, 1A) from the environment during the formation and the following healing of the disruption in the cell membrane (2).

10. The method according to claim 8 or 9, in which the cell membrane (2) has another form, composition and/or size after the healing than before the formation of the disruption.

11. The method according to claim 10, in which the cell (1, 1A) is separated after the healing into two cellular bodies (4, 5) that each have a topologically closed form.

12. The method according to claim 9, in which one of the separated bodies (5) is without a nucleus.

13. The method according to claim 12, in which the part (5) without the nucleus is subjected to an investigation.

14. The method according to claim 10, in which the cell membrane (2) forms at least one topologically closed cell body with a foreign cell membrane (2A) of another biological cell (1A) after the healing.

15. The method according to claim 10, in which the biological cell (1) is fused with at least one other biological cell (1A) to a fusion cell (6).

16. The method according to claim 15, in which the cells (1, 1A) are arranged superposed above one another on a substrate (20) during the fusion.

17. A cell manipulator (100), especially for treating biological cells (1, 1A), that comprises:
- a substrate (20) for receiving a cell (1, 1A),
- a tool (10, 10A) for treating the biological cell (1, 1A), and
- a drive device (30, 30A) for moving the tool (10, 10A) relative to the substrate (20),
**characterized in that**
- the tool (10, 10A) has a work surface (11) under the action of which the cell membrane (2) of the biological cell (1, 1A) can be locally selectively deformed, wherein
- the work surface (11) of the tool (10, 10A) has a characteristic size that is less than 10 µm.

18. The cell manipulator according to Claim 17, in which the work surface (11) of the tool (10, 10A) carries a coating (12) that hinders an adhesion of cells.

19. The cell manipulator according to at least one of Claims 17 or 18, in which the drive device (30, 30A) is adapted to move the tool (10, 10A) at rates in a range of 0.1 µm/h to 500 µm/h.

20. The cell manipulator according to at least one of Claims 17 to 19, in which the drive device (30, 30A) has a piezoelectric drive or a magnetic drive.

21. The cell manipulator according to at least one of Claims 17 to 20, that has a positioning device (40) for positioning the drive device (30, 30A) and/or a substrate (20) with the biological cell (1, 1A).

22. The cell manipulator according to at least one of Claims 17 to 21, that has a sensor device (50) for detecting the position of the tool (10, 10A) and/or of the biological cell (1, 1A).

23. The cell manipulator according to at least one of Claims 17 to 22, that has at least two tools (10, 10A).

24. The cell manipulator according to at least one of Claims 17 to 23, in which the tool (10, 10A) has a straight side edge (12) or structured side edge sections (12A).

25. The use of a method or of a cell manipulator according to at least one of the preceding claims for the:
- fusion of biological cells, and/or
- diagnosis or characterization of individual cells.

## Revendications

1. Procédé de traitement d'une cellule biologique (1, 1A) qui comprend un cytosquelette (3) enveloppé d'une membrane plasmique (2), comportant les étapes :
- orientation réciproque de la cellule biologique (1, 1A) et d'un outil (10, 10A), de telle sorte que l'outil (10, 10A) touche la cellule biologique (1, 1A), et
- déplacement de l'outil (10, 10A) et de la cellule biologique (1, 1A) l'un par rapport à l'autre,
**caractérisé en ce que**
- avec l'outil (10, 10A) est réalisée une scission dans la liaison moléculaire de la membrane plasmique (2) de la cellule biologique (1, 1A), et
- le déplacement de l'outil (10, 10A) et de la cellule biologique (1, 1A) l'un par rapport à l'autre est effectué lentement, de telle sorte que le cytosquelette et la membrane plasmique de la cellule biologique (1, 1A) peuvent être ajustés à tout moment aux conditions géométriques données par une surface de travail de l'outil (10, 10A), de telle sorte que pendant le déplacement de l'outil (10, 10A), le cytosquelette (3) de la cellule biologique (1, 1A) possède un état d'équilibre.

2. Procédé selon la revendication 1, dans lequel le déplacement de l'outil (10, 10A) est effectué à une vitesse inférieure ou égale à une vitesse de réorganisation du cytosquelette (3).

3. Procédé selon la revendication 1 ou 2, dans lequel le déplacement de l'outil (10, 10A) est effectué à une vitesse inférieure ou égale à une vitesse de migration des cellules adhérentes sur une surface.

4. Procédé selon la revendication 2 ou 3, dans lequel le déplacement de l'outil (10, 10A) est effectué à une vitesse inférieure à 500 µm/h.

5. Procédé selon l'une quelconque des revendications précédentes, dans lequel la cellule biologique (1, 1A) est disposée sur un substrat (20), et l'outil (10, 10A) est déplacé par rapport au substrat (20).

6. Procédé selon la revendication 5, dans lequel le déplacement de l'outil (10, 10A) par rapport au substrat (20) comprend une phase d'approche, dans laquelle la distance entre l'outil (10, 10A) et le substrat (20) est diminuée, et/ou une phase de translation, dans laquelle l'outil (10, 10A) est déplacé parallèlement à la surface du substrat (20).

7. Procédé selon au moins l'une quelconque des revendications précédentes, dans lequel, pendant le déplacement, la membrane plasmique (2) est soumise par l'outil (10, 10A) à une déformation, un écrasement et/ou un allongement.

8. Procédé selon au moins l'une quelconque des revendications précédentes, comportant l'étape suivants :
- cicatrisation de la scission, de telle sorte que la membrane plasmique (2) se ferme, la cicatrisation comprenant une cicatrisation spontanée, pendant laquelle la membrane plasmique se ferme sous l'effet de la tension superficielle en présence de l'outil au repos, ou la cicatrisation étant favorisée par un déplacement dirigé de l'outil.

9. Procédé selon la revendication 8, dans lequel, pendant la formation et la cicatrisation consécutive de la scission de la membrane plasmique (2), il se produit une séparation entre un volume intérieur de la cellule (1, 1A) et l'environnement.

10. Procédé selon la revendication 8 ou 9, dans lequel la membrane plasmique (2) après la cicatrisation possède une forme, composition et/ou taille différentes de celles avant la formation de la scission.

11. Procédé selon la revendication 10, dans lequel la cellule (1, 1A) est séparée après la cicatrisation en deux corps cellulaires (4, 5), qui présentent chacun une forme fermée sur le plan topologique.

12. Procédé selon la revendication 9, dans lequel l'un des corps cellulaires (5) séparés est un corps anucléé.

13. Procédé selon la revendication 12, dans lequel le corps anucléé (5) est soumis à un examen.

14. Procédé selon la revendication 10, dans lequel la membrane plasmique (2) après la cicatrisation forme avec une membrane plasmique (2A) étrangère d'une autre cellule biologique (1A) au moins un corps cellulaire fermé sur le plan topologique.

15. Procédé selon la revendication 10, dans lequel la cellule biologique (1) est fusionnée avec au moins une autre cellule biologique (1A) pour obtenir une cellule fusionnée (6).

16. Procédé selon la revendication 15, dans lequel, pendant la fusion, les cellules (1, 1A) sont disposées l'une au-dessus de l'autre sur un substrat (20).

17. Manipulateur de cellules (100), en particulier pour le traitement de cellules biologiques (1, 1A), lequel comprend :
- un substrat (20) destiné à recevoir une cellule (1, 1A),
- un outil (10, 10A) pour le traitement de la cellule biologique (1, 1A), et
- un dispositif d'entraînement (30, 30A) pour le déplacement de l'outil (10, 10A) par rapport au substrat (20), **caractérisé en ce que**
- l'outil (10, 10A) comporte une surface de travail (11), sous l'action de laquelle la membrane plasmique (2) de la cellule biologique (1, 1A) est déformable localement de manière sélective,
- la surface de travail (11) de l'outil (10, 10A) ayant une grandeur caractéristique qui est inférieure à 10 µm.

18. Manipulateur de cellules selon la revendication 17, dans lequel la surface de travail (11) de l'outil (10, 10A) porte un revêtement (12) qui empêche l'adhérence des cellules.

19. Manipulateur de cellules selon au moins une des revendications 17 ou 18, dans lequel le dispositif d'entraînement (30, 30A) est configuré pour déplacer l'outil (10, 10A) à des vitesses dans une plage de 0,1 µm/h à 500 µm/h.

20. Manipulateur de cellules selon au moins l'une quelconque des revendications 17 à 19, dans lequel le dispositif d'entraînement (30, 30A) comporte un entraînement piézoélectrique ou un entraînement magnétique.

21. Manipulateur de cellules selon au moins l'une quelconque des revendications 17 à 20, lequel comporte un dispositif de positionnement (40) pour le positionnement du dispositif d'entraînement (30, 30A) et/ou d'un substrat (20) avec la cellule biologique (1, 1A).

22. Manipulateur de cellules selon au moins l'une quelconque des revendications 17 à 21, lequel comporte un dispositif de détection (50) destiné à détecter la position de l'outil (10, 10A) et/ou de la cellule biologique (1, 1A).

23. Manipulateur de cellules selon au moins l'une quelconque des revendications 17 à 22, lequel comporte au moins deux outils (10, 10A).

24. Manipulateur de cellules selon au moins l'une quelconque des revendications 17 à 23, dans lequel l'outil (10, 10A) comporte un bord latéral (12) droit ou des portions de bord latéral (12A) structurées.

25. Utilisation d'un procédé ou d'un manipulateur de cellules selon au moins l'une quelconque des revendications précédentes, pour :
- la fusion de cellules biologiques, et/ou
- le diagnostic ou la caractérisation de cellules individuelles.
